# EUROPEAN PATENT APPLICATION

(11) **EP 2 589 408 A2**
(43) Date of publication of application: **08.05.2013**
(21) Application number: 13153900.9
(22) Date of filing: 28.01.2011
(51) Int. Cl.: A61M 37/00

(54) **Micro needle and micro needle apparatus**

(30) Priority: 29.01.2010 KR 20100008306; 11.05.2010 KR 20100043711; 14.09.2010 KR 20100090187
(62) Divisional of application: 11737313.4
(71) Applicant: Ubiomed Inc., Buk-Gu, Daegu 702-701 (KR)
(72) Inventor: Eum, Nyeon Sik, 702-040 Daegu (KR)
(74) Representative: Epping - Hermann - Fischer

(57) **Abstract**

The micro needle includes a needle (1) and a body (2). The needle includes an inclined part (10) including an inclined outer wall, a straight part (20) including a straight outer wall, and a recess (31) having a certain depth along the inclined or straight outer wall. The body is coupled to the needle to move or support the needle.

## Description

### TECHNICAL FIELD

The present disclosure relates to a micro needle and a micro needle apparatus, and more particularly, to a micro needle having improved medication transferring efficiency and resistant to a breakage, and a micro needle apparatus for more efficiently injecting a vaccine for active immunity or a skin elasticity treatment for skin care into a human body through the skin, and maximizing bioactive effect of an infrared ray.

### BACKGROUND ART

Medicines or medications for active immunity such as treatments or vaccines (hereinafter, referred to as medications) are transferred into a human body through an oral administration or an injection. However, when medications are transferred through an oral administration, operations of digestive enzymes within an alimentary canal may prevent the medications from being directly absorbed into a blood flow, or may modify the medications so as to jeopardize desired effects thereof. When medications are transferred through an injection, they are directly absorbed into a blood flow through the skin or a vein. Accordingly, unlike through an oral administration, the medications are prevented from being modified by digestive enzymes within an alimentary canal. However, a needle penetrates the dermis of the skin during the injection, which may cause pain or infection.

To address these limitations of injections and oral administrations, percutaneous methods for transferring a medication to a local tissue or the whole circulatory system through the skin are actively researched.

Such a percutaneous method may be used with, e.g., a micro need apparatus including a micro needle head having a plurality of micro needles. The micro needles of the micro need apparatus make it possible to inject a medication through a skin without pain, and physically penetrate the epidermis including the corneum of the skin so as to improve spread speed of the medication from a medication supply part to the dermis.

When typical patternless micro needles are used to inject a medication, the medication is transferred to a skin along smooth outer walls of the patternless micro needles. However, at this point, a large amount of the medication is blocked by the skin, and a very small amount of the medication is injected into the skin.

In addition, when hollow-type needles having a hollow part in the center thereof are used to inject a medication to a skin, they are susceptible to a breakage, and air from the hollow part may be introduced to the skin, before the injection. Furthermore, typical micro needle apparatuses do not include a device for relaxing a skin having pores constricted at low temperature, or a hardened cell tissue. Thus, when a medication is injected into the epidermis including the corneum of a skin through micro needles at low temperature or in winter, absorption efficiency of the medication through the epidermis, or transfer efficiency of the medication into the dermis may be degraded. As a result, injection efficiency of the medication may be degraded.

Furthermore, typical micro needle apparatuses do not have a device that prevents leakage of a medication to be supplied micro needles through a medication supply part before or after the medication is injected from a micro needle head. Thus, when the micro needle apparatus is moved to use or store the micro needle apparatus before or after the medication is injected, or when the micro needle head is shaken, the medication may leak through the micro needles.

In addition, when pores of a skin are constricted at low temperature, or a cell tissue is hardened, performances of typical micro needle apparatuses are significantly degraded. That is, even though micro needles penetrate the epidermis including the corneum of a skin at low temperature or in winter, absorption efficiency of a medication through the epidermis, or transfer efficiency of the medication into the dermis may be degraded so as to decrease natural healing efficiency of cells of the dermis.

To address these limitations, a device is suggested for relaxing a skin by using a heating operation of infrared rays, to thereby improve performances of micro needles. However, such devices using infrared rays within a typical micro needle apparatus do not effectively perform other operations of the infrared rays than the heating operation.

### DISCLOSURE OF THE INVENTION

### TECHNICAL PROBLEM

Embodiments provide a micro needle for injecting a medication into a skin through a recess disposed in the outer wall of the micro needle, thereby improving medication transferring efficiency of the micro needle, and preventing a breakage thereof.

Embodiments also provide a micro needle apparatus that heats a skin to expand pores thereof when the medication is injected, or stimulates the skin by an infrared ray and/or a far infrared ray to medication transferring efficiency. Embodiments also provide a micro needle apparatus that emits an infrared ray to the inside of a skin as well as the epidermis, thereby maximizing effects of the infrared ray.

### TECHNICAL SOLUTION

In one embodiment, a micro needle includes: a needle including an inclined part including an inclined outer wall, a straight part including a straight outer wall, and a recess having a certain depth along the inclined or straight outer wall; and a body coupled to the needle to move or support the needle. Thus, the micro needle has improved medication transferring efficiency, and is resistant to a breakage.

In another embodiment, a micro needle apparatus includes: a medication storage part for storing a medication; a micro needle head including a medication passage connected to the medication storage part, and a plurality of micro needles having front ends protruding out of the micro needle head, wherein, when the micro needles are pressed against a skin, the micro needles penetrate a dermis of the skin to transfer the medication from the medication passage to the dermis of the skin; and a heating part for heating the skin through the micro needle head.

In another embodiment, a micro needle apparatus includes: a micro needle head part including a plurality of micro needles having front ends protruding out of the micro needle head part, wherein, when the micro needles are pressed against a skin, the micro needles arrive at a dermis of the skin; an infrared generator part generating an infrared or far infrared ray to the skin through an inside or outside of the micro needle; and a power supply part for supplying energy required for the infrared generator part to generate the infrared or far infrared ray.

### ADVANTAGEOUS EFFECTS

The micro needle according to the various embodiments attains the following effects.

According to the first embodiment, since the needle has a cross (+) shape, and a medication can be transferred into a skin. In addition, since the recesses are disposed only in the side wall of the needle, the strength of the needle can be ensured, to thereby prevent the needle from being broken within a skin when a medication is injected.

According to the second embodiment, since the needle includes the recess having a cross (+) shape, a certain volume of a medication is captured within the recess, and is injected into a skin. Thus, the medication captured within the recess can be effectively injected into the skin. In addition, since the recess is short within the straight part of the needle, the strength of the needle can be ensured, to thereby prevent the needle from being broken within a skin when a medication is injected.

According to the third embodiment, since the recess extending along the central portion of the needle has a large volume, a larger amount of medication can be efficiently transferred into a skin.

According to the fourth embodiment, since the needle includes the recess having a flat (-) shape, a certain volume of a medication is captured within the recess, and is injected into a skin. Thus, the medication captured within the recess can be effectively injected into the skin. In addition, since the recess is short within the straight part of the needle, the strength of the needle can be higher than that of the second embodiment, to thereby fundamentally prevent the needle from being broken within a skin when a medication is injected.

According to the fifth embodiment, since the two small needles face each other, a larger amount of medication can be captured than that of the fourth embodiment. In addition, since the needle constituted by the two small needles includes the recess having a flat (-) shape, the strength of the needle can be further increased, to thereby prevent the needle from being broken within a skin when a medication is injected.

According to the sixth embodiment, the needle includes the recess having a screw thread shape to capture a certain volume of a medication. In addition, when the needle is inserted into a skin, a medication from the body can be circumferentially rotated along the recess having a screw thread shape, and be slowly injected into the skin.

Thus, according to the above-described various embodiments, a medication is injected into a skin through the recess disposed in the outer wall of the micro needle, thereby improving the medication transferring efficiency of the micro needle, and preventing a breakage thereof.

In addition, the micro needle apparatus according to the embodiments includes: the heater for heating a skin to expand pores thereof when a medication is injected; and/or the infrared generator part and/or the far infrared generator part to stimulate the skin by an infrared ray and/or a far infrared ray. Thus, when a medication is injected, the micro needle apparatus excites skin cells by heat from the heater and an infrared ray and/or a far infrared ray from the infrared generator part and/or the far infrared generator part, thereby activating the cells and obtaining improved blood circulation within the skin, thermotherapy effect, ripening effect, dryness-and-moisture effect, vaporization effect, and resonance effect. Accordingly, the medication passes through the epidermis of the skin along the micro needles, and arrives at the dermis through expanded pores and the activated cells, thereby improving the medication transferring efficiency.

In addition, the medication storage part according to the embodiments is formed by connecting the medication storage chamber and the medication supply tube to each other, and the medication storage chamber includes the medication storage, the medication chamber, the medication selector, and at least two medication cells that are longitudinally separated to receive various medications. Thus, various medications can be simply and selectively supplied from the medication storage part to a skin by the medication selector.

In addition, the medication blocking part opens the medication passage when the micro needle head contacts a skin, and the medication blocking part closes the medication passage when the micro needle head is spaced apart from the skin. Thus, when the micro needle head is spaced apart from the skin before or after a medication is injected, the medication is automatically prevented from leaking through the micro needles, so that a user can freely move the micro needle head to a target position without worrying about leaking of the medication from the micro needle head.

In addition, since the micro needle apparatus according to another embodiment emits infrared rays from the outside of a skin, and supplies the infrared rays to the inside of the skin, so that the skin improving effect of the micro needles and the bioactive operation of the infrared rays can be very efficiently combined. Specifically, when the micro needle apparatus is used for a human head, the scalp can be stimulated by the micro needles and infrared rays, and follicles of the scalp can be stimulated and heated to improve hair quality and prevent hair loss.

### BRIEF DESCRIPTION OF THE DRAWINGS

Figs. 1A to 1D are views illustrating a micro needle according to a first embodiment.
Figs. 2A to 2D are views illustrating a micro needle according to a second embodiment.
Figs. 3A to 3D are views illustrating a micro needle according to a third embodiment.
Figs. 4A to 4D are views illustrating a micro needle according to a fourth embodiment.
Figs. 5A to 5D are views illustrating a micro needle according to a fifth embodiment.
Figs. 6A to 6D are views illustrating a micro needle according to a sixth embodiment.
Fig. 7 is a perspective view illustrating a micro needle apparatus according to an embodiment.
Fig. 8 is an exploded perspective view illustrating a micro needle apparatus according to an embodiment.
Fig. 9 is a cross-sectional view illustrating a medication storage chamber of a medication storage part of a micro needle apparatus according to an embodiment.
Fig. 10 is a cross-sectional view taken along line 2-2 of
Fig. 9.

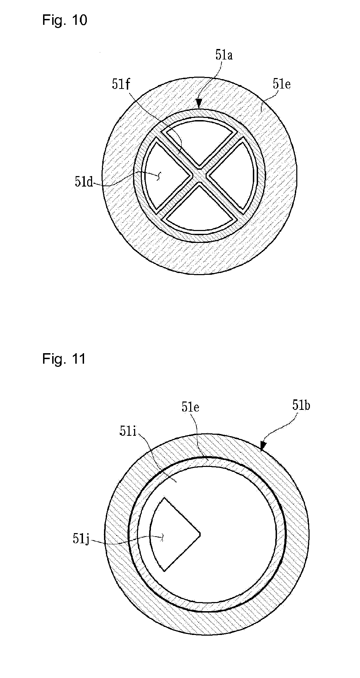

Fig. 11 is a cross-sectional view taken along line 3-3 of
Fig. 9.

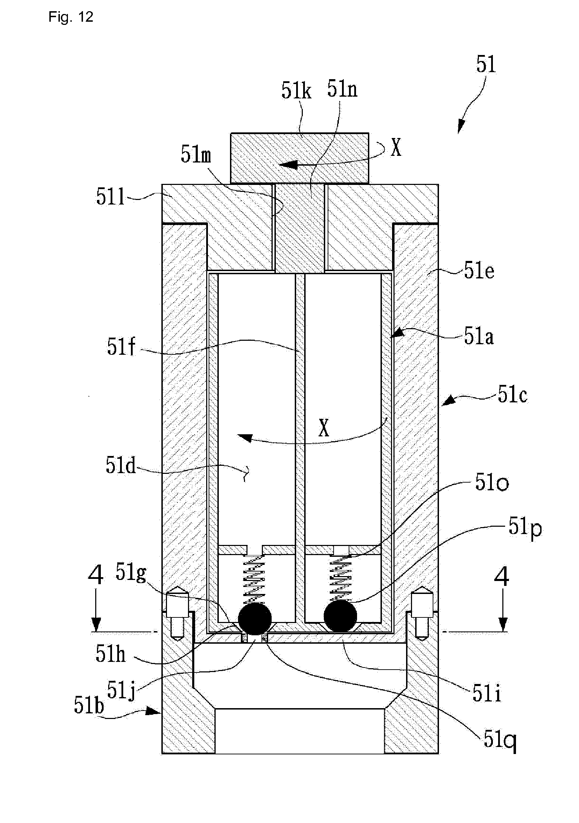

Fig. 12 is a cross-sectional view illustrating a medication storage chamber of a medication storage part of a micro needle apparatus according to an embodiment.

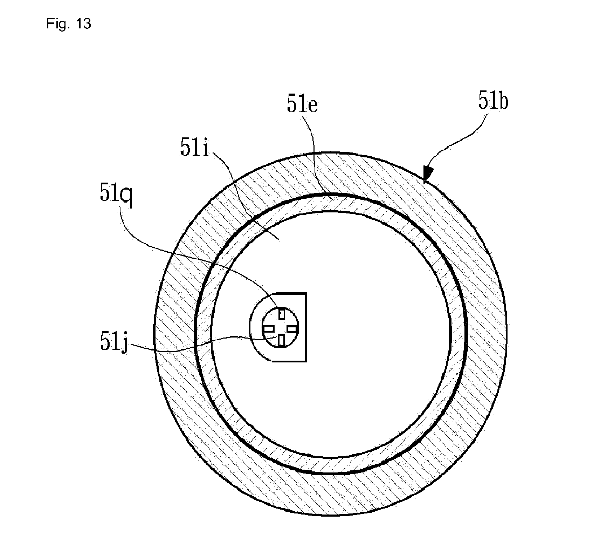

Fig. 13 is a cross-sectional view taken along line 4-4 of
Fig. 12.

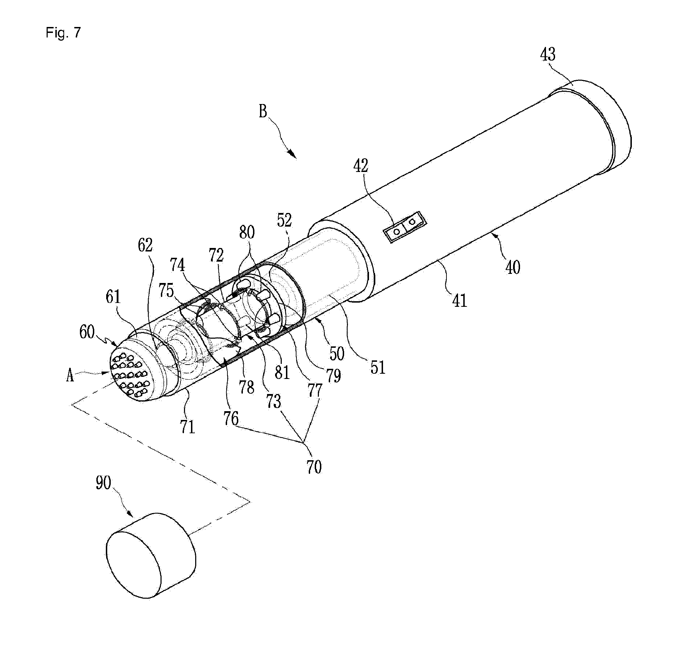

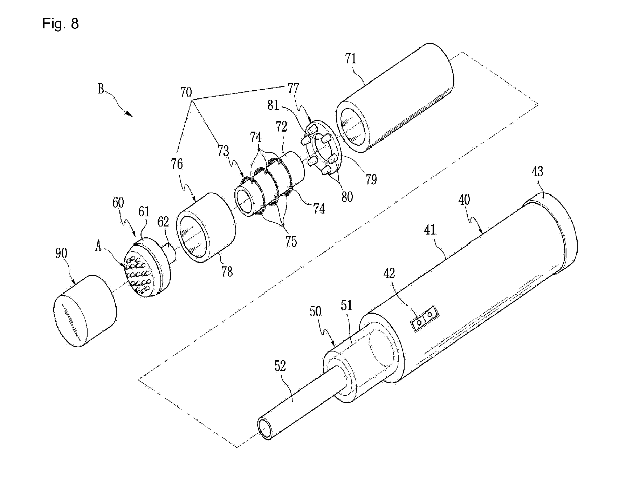

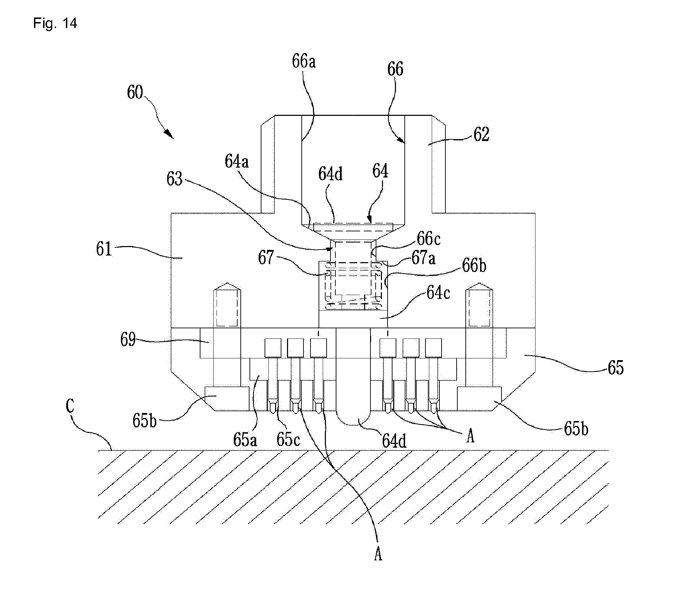

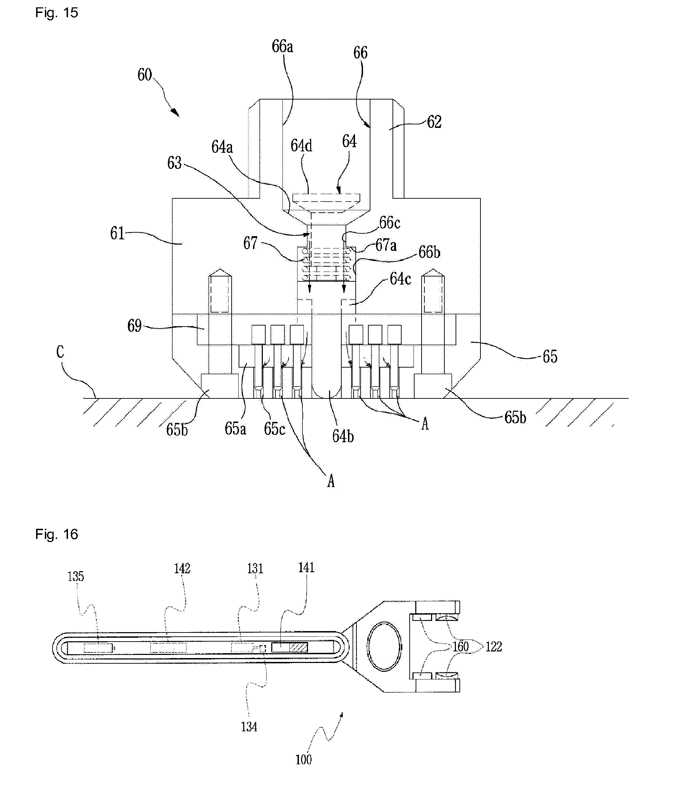

Figs. 14 and 15 are cross-sectional views illustrating an operation of the micro needle apparatuses of Figs. 7 and 8.
Fig. 16 is a cross-sectional view illustrating a micro needle roller in the relate art.

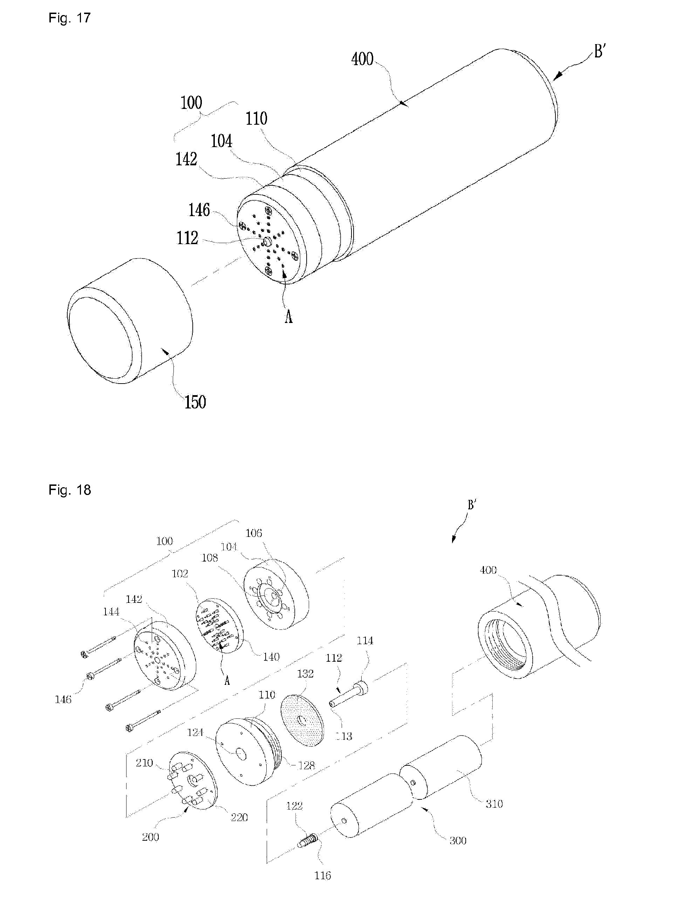

Fig. 17 is a perspective view illustrating a micro needle apparatus according to an embodiment.
Fig. 18 is an exploded perspective view illustrating a micro needle apparatus according to an embodiment.

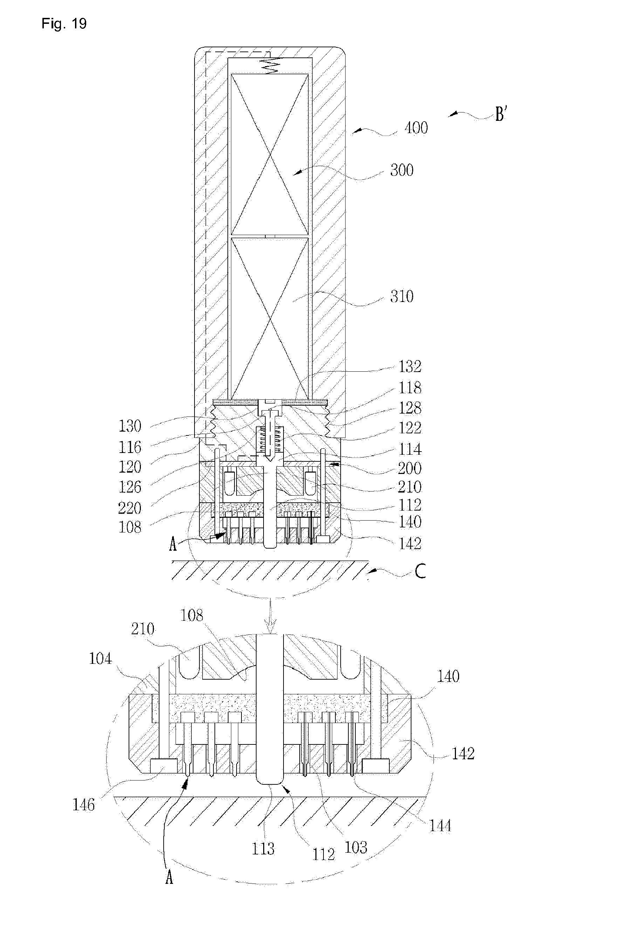

Fig. 19 is a cross-sectional view illustrating a switch body of a micro needle apparatus in a second position according to an embodiment.

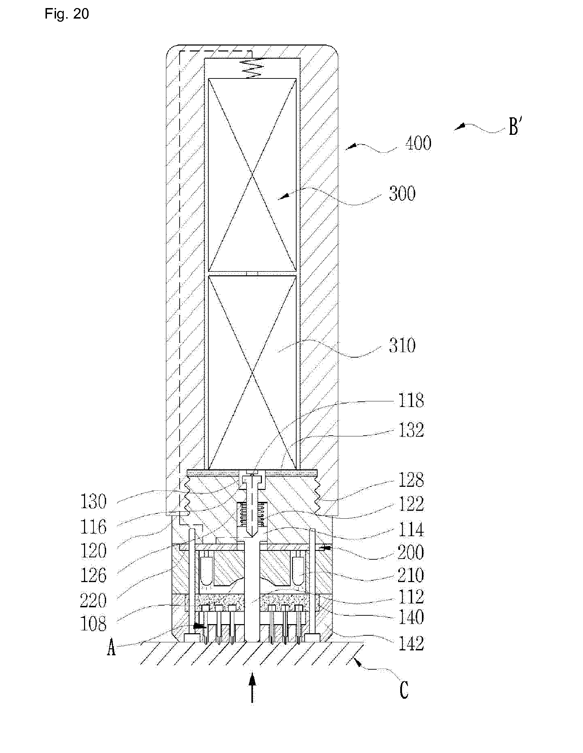

Fig. 20 is a cross-sectional view illustrating a switch body of a micro needle apparatus in a first position according to an embodiment.

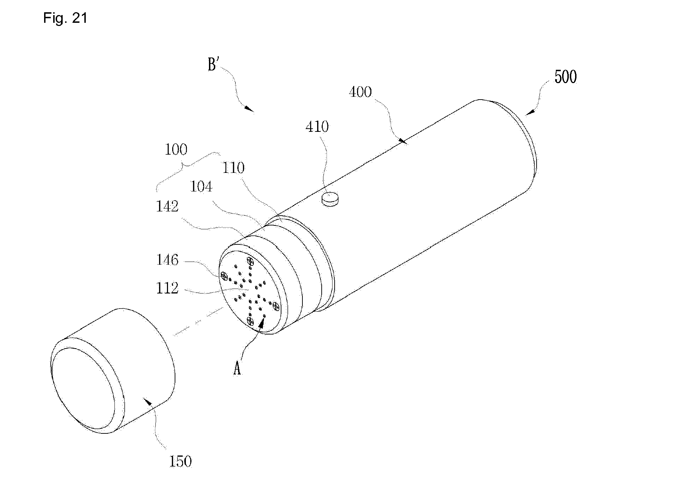

Fig. 21 is a perspective view illustrating a micro needle apparatus according to an embodiment.

### MODE FOR CARRYING OUT THE INVENTION

Hereinafter, a micro needle according to embodiments will now be described in detail with reference to the accompanying drawings.

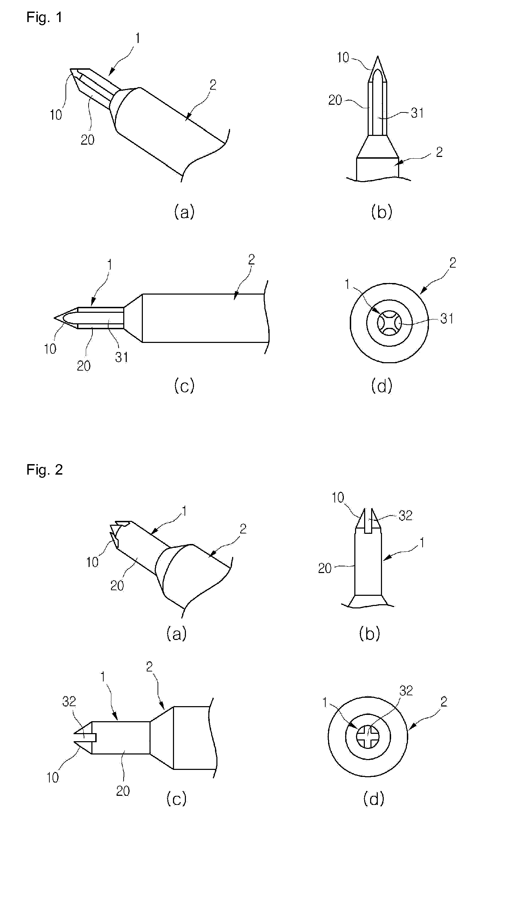

Figs. 1A to 6D are views illustrating a micro needle A according to embodiments, in which: the drawings corresponding to the alphabetic character A are perspective views; the drawings corresponding to the alphabetic character B are front views; the drawings corresponding to the alphabetic character C are side views; and the drawings corresponding to the alphabetic character D are plan views. Referring to Fig. 1, a micro needle according to a first embodiment includes a needle 1 and a body 2.

The needle 1 penetrates a skin to inject a medication. The body 2 is coupled to the needle 1 to move or support the needle 1. A medication is transferred from the body 2 to the needle 1. The body 2 has an approximately cylindrical shape and a taper shape that decreases in diameter at a portion connecting to the needle 1 having a diameter smaller than that of the body 2.

The needle 1 includes: a straight part 20 having an end contacting the body 2, and including a straight outer wall; and an inclined part 10 extending outward from the other end of the straight part 20, and including an inclined outer wall with a sharp end decreasing in diameter.

Both the inclined part 10 and the straight part 20 may penetrate a skin, or only the inclined part 10 may penetrate a skin according to a user's need.

Recesses 31 having a straight line shape extending along the needle 1, and recessed a certain depth toward the center of the needle 1 are disposed in the outer wall of the inclined part 10 and the outer wall of the straight part 20. The recess 31 extends from an end of the straight part 20 through the other end thereof to the inclined part 10, and may be provided in four arrayed with a constant interval on the upper, lower, left, and right sides of the outer wall of the needle 1 as illustrated in Figs. 1D. Alternatively, the recess 31 may be provided in three or more.

When the recess 31 is provided in four, the needle 1 has a cross (+) shape as illustrated in Fig. 1D. Thus, a medication can be efficiently transferred into a skin along the recesses 31 disposed in the side wall of the needle 1. In addition, since the recesses 31 are disposed only in the side wall of the needle 1, the strength of the needle 1 can be ensured, to thereby prevent the needle 1 from being broken within a skin when a medication is injected.

Figs. 2A to 2D are views illustrating a micro needle according to a second embodiment. Like reference numerals denote like elements in the first and second embodiments, and thus, a description thereof will be omitted in the current embodiment.

Referring to Figs. 2A to 2D, a cross-shaped recess 32 is recessed a certain depth toward a body 2 in the central portion of an inclined part 10 of a needle 1. The recess 32 may extend from an end of the inclined part 10 to a portion of a straight part 20 as illustrated in Figs. 2B and 2C, and have a cross (+) shape with four branches toward the upper, lower, left, and right sides of the needle 1 as illustrated in Fig. 2D. Although not shown, from a plan view, the recess 32 may have a shape with six branches such as an asterisk (*), or a shape with three or more braches.

Since the recess 32 has a cross (+) shape as illustrated in Figs. 2A to 2D, a certain volume of a medication is captured within the recess 32. In this state, when the needle 1 is inserted into a skin, the medication captured within the recess 32 is effectively injected into the skin. In addition, since the recess 32 has a short portion in the straight part 20 of the needle 1, the strength of the needle 1 can be ensured, to thereby prevent the needle 1 from being broken within a skin when a medication is injected.

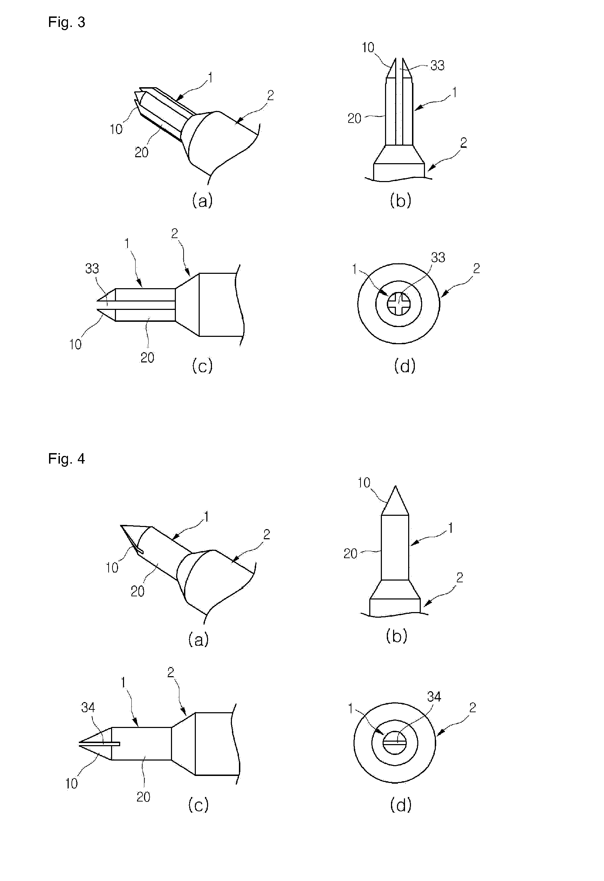

Figs. 3A to 3D are views illustrating a micro needle according to a third embodiment, which is a modification of the second embodiment of Figs. 2A to 2D. Referring to Fig. 3D, a recess 33 is the same as the recess 32 of Figs. 2A to 2D in a cross (+) shape with four branches toward the upper, lower, left, and right sides, or a shape with at least five branches.

However, as illustrated in Figs. 3A to 3C, the recess 33 entirely extends through not only an inclined part 10, but also a straight part 20. Accordingly, the needle 1 is divided into four small sharp needles, so that a larger amount of a medication can be captured within the recess 33 disposed between the small sharp needles. Although the strength of the needle 1 of the third embodiment is slightly lower than that of the needle 1 of the second embodiment, the volume of the recess 33 extending along the central portion of the needle 1 is increased, so that a larger amount of a medication can be efficiently injected into a skin.

Figs. 4A to 4D are views illustrating a micro needle according to a fourth embodiment. Referring to Figs. 4A to 4D, a flat-shaped (-) recess 34 is recessed a certain depth toward a body 2 in the central portion of an inclined part 10 of a needle 1. The recess 34 may extend from an end of the inclined part 10 to a portion of a straight part 20 as illustrated in Fig. 4C, and have a flat (-) shape with two branches toward the left and right (or upper and lower) sides of the needle 1 as illustrated in Fig. 4D.

Since the recess 34 has a flat (-) shape as illustrated in Figs. 4A to 4D, a certain volume of a medication is captured within the recess 34. In this state, when the needle 1 is inserted into a skin, the medication captured within the recess 34 is effectively injected into the skin. In addition, since the recess 34 has a short portion in the straight part 20 of the needle 1, and the branches of the recess 34 are fewer than those of the recess 33 of the second embodiment, the strength of the needle 1 can be increased, to thereby fundamentally prevent the needle 1 from being broken within a skin when a medication is injected.

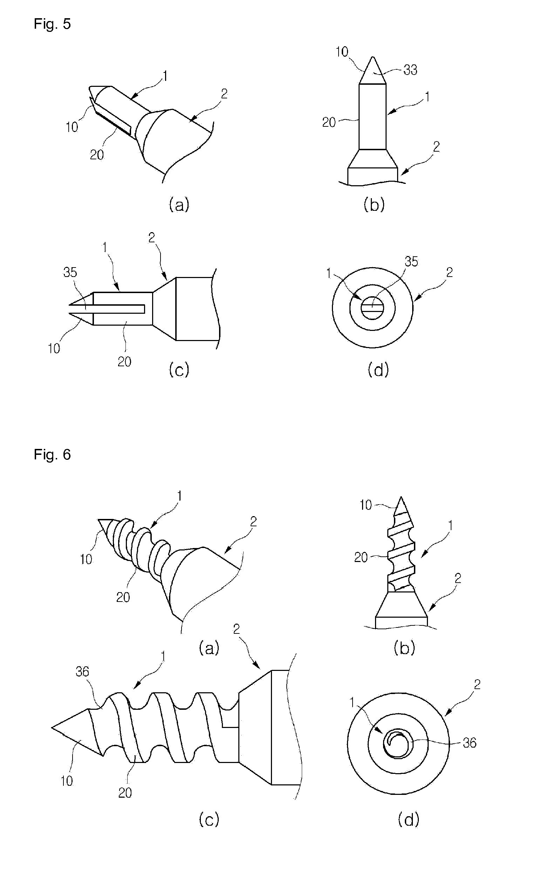

Figs. 5A to 5D are views illustrating a micro needle according to a fifth embodiment, which is a modification of the fourth embodiment of Figs. 4A to 4D. Referring to Fig. 5D, a recess 35 is the same as the recess 34 of Figs. 4A to 4D in a flat (-) shape with two branches toward the left and right (or upper and lower) sides.

However, as illustrated in Figs. 5A and 5C, the recess 35 entirely extends through not only an inclined part 10, but also a straight part 20. Accordingly, the needle 1 is divided into two small face-to-face sharp needles, so that a larger amount of a medication can be captured within the recess 35 disposed between the two small sharp needles than the amount of a medication captured within the recess 34 of the fourth embodiment. Although the strength of the needle 1 of the fifth embodiment is slightly lower than that of the needle 1 of the fourth embodiment, the volume of the recess 35 extending along the central portion of the needle 1 is increased, so that a larger amount of a medication can be efficiently injected into a skin. In addition, since the recess 35 has a flat (-) shape, unlike the recess 33 of the third embodiment, the strength of the needle 1 can be further increased, to thereby prevent the needle 1 from being broken when a medication is injected.

Figs. 6A to 6D are views illustrating a micro needle according to a sixth embodiment. Referring to Fig. 6, a recess 36 having a screw thread shape with a certain depth extends along the outer wall of a needle 1. The recess 36 is disposed on the whole area of a straight part 20 of the needle 1, and a portion of an inclined part 10. The recess 36 may rotate clockwise as illustrated in Fig. 6D, or rotate counterclockwise, and have a curved inner surface, and the width thereof may be varied according to a design.

Thus, according to the sixth embodiment, the recess 36 can capture a certain volume of a medication. In addition, when the needle 1 is inserted into a skin, a medication from a body 2 can be circumferentially rotated along the recess 36, and be slowly injected into the skin.

According to the above-described various embodiments, a medication is injected into a skin through a recess disposed in the outer wall of a micro needle, thereby improving medication transferring efficiency of the micro needle, and preventing a breakage thereof.

Although a medication is transferred into a skin through the micro needle in the embodiments, a cosmetic can be transferred through the micro needle.

Hereinafter, a micro needle according to embodiments will now be described in detail with reference to the accompanying drawings.

Figs. 7 and 8 are perspective views illustrating a micro needle apparatus B according to an embodiment.

The micro needle apparatus B is a percutaneous apparatus for more efficiently injecting a vaccine for active immunity or a skin elasticity treatment for skin care into a body through a skin. The micro needle apparatus B includes a power source part 40, a medication storage part 50, a micro needle head 60, and a heating part 70.

The power source part 40 includes a barrel body 41 having a cylindrical shape. The barrel body 41 has a battery space for accommodating a dry cell or a battery. For example, two 1.5V dry cells may be arrayed in series within the battery space. A switch 42 for selectively supplying power is disposed on the outer circumferential surface of the barrel body 41, and a cover 43 closes the bottom of the barrel body 41. The barrel body 41 not only functions as the power source part 40 for accommodating a dry cell or a battery but also functions as a body for a user to hold the micro needle apparatus B when injecting a medication.

The medication storage part 50 couples to the barrel body 41 of the power source part 40, and includes a medication storage chamber 51 therein to store a medication. A medication supply tube 52 is disposed at an outlet of the medication storage chamber 51. The medication supply tube 52 extends and air-tightly connects to an inlet 62 of a fixing body 61 of the micro needle head 60 to be described later. The heating part 70 is disposed between the medication storage part 50 and the micro needle head 60. When a medication is injected into a skin, the heating part 70 heats the skin to expand pores, and stimulates the skin to activate skin cells. To this end, the heating part 70 includes an outer barrel 71, an inner barrel 72, and a heater 73 disposed between the outer barrel 71 and the inner barrel 72.

The right end of the outer barrel 71 is coupled to the left end of the medication storage part 50 by a member such as a screw. The outer barrel 71 includes an insulator for blocking heat. The inner barrel 72 receives and passes the medication supply tube 53. Like the outer barrel 71, the inner barrel 72 includes an insulator for protecting the medication supply tube 53 from heat emitted from the heater 73. Supports 74 installed on the outer portion of the inner barrel 71 fix the heater 73 in a position spaced a certain distance from the micro needle head 60. The heater 73 heats the micro needle head 60 to transmit heat to a skin. For example, the heater 73 includes paratactically connected heating wires 75, such as Nichrome wires having a width of about 3 mm, to continually emit heat ranging from about 40 to 50 °C.

As described above, the heater 73 is fixed in the position spaced a certain distance from the micro needle head 60, and indirectly heats the micro needle head 60. Alternatively, the heater 73 may directly contact the micro needle head 60 and/or micro needles A of the micro needle head 60 to directly heat the micro needle head 60 and/or the micro needles A to transmit heat to a skin.

Far infrared rays and/or infrared rays may be emitted on a skin to stimulate the skin, so that the micro needles A of the micro needle head 60 to be described later can more efficiently transfer a medication to the dermis of the skin. To this end, the heating part 70 may include a far infrared generator 76 and/or an infrared generator 77.

Although the micro needles A are exemplified in the current embodiment, the present disclosure is not limited thereto, and thus, typical needles that have no screw thread or recess around the side wall thereof may also be used.

The far infrared generator 76 is coupled to the inner portion of the outer barrel 71 to face the heater 73, so that far infrared rays can be generated by heat from the heater 73. To this end, the far infrared generator 76 includes a cylinder 78 that has an outer diameter smaller than the inner diameter of the outer barrel 71, and that is disposed on the outer portion of the heater 73 within the left portion of the outer barrel 71. The cylinder 78 may be formed of a gem stone or ceramic.

Alternatively, the far infrared generator 76 may be disposed between the heater 73 and the inner barrel 72. In this case, the far infrared generator 76 includes a cylinder (not shown) having an inner diameter greater than the outer diameter of the inner barrel 72, and the heater 73 is fixed by supports (not shown) installed on the outer portion of the cylinder. The infrared generator 77 is disposed between the heater 73 and the medication storage part 50 to generate infrared rays. The infrared generator 77 includes a fixing ring 79 having a circular shape, and a plurality of infrared LEDs or lamps 80 to generate infrared rays having a wavelength, e.g., ranging from about 700 nm to 20 µm. The fixing ring 79 includes a circular hole 81 in the central portion thereof, and the inner barrel 72 is fitted in the circular hole 81. Far infrared rays and infrared rays generated from the far infrared generator 76 and the infrared lamps 80, and heat generated from the heater 73 are reflected to the micro needle head 60. To this end, the surface of the fixing ring 79 where the infrared lamps 80 are installed may include a reflective surface. The infrared lamps 80 are arrayed with a certain interval around the circular hole 81 of the fixing ring 79.

The heater 73 and the infrared lamps 80 are electrically connected to the power source part 40 through wires (not shown) disposed in the medication storage part 50.

As such, far infrared rays generated from the far infrared generator 76, and infrared rays generated from the infrared generator 77 may have the following effects: skin cells can be excited and activated during an injection; active oxygen accumulated by environment pollution can be removed from the body; double bonds of unsaturated fatty acid can be caused to improve cosmetic effect; and a tissue acidified by an inflammation can be alkalized. Accordingly, improved blood circulation within the skin, thermotherapy effect, ripening effect, dryness-and-moisture effect, vaporization effect, and resonance effect can be obtained.

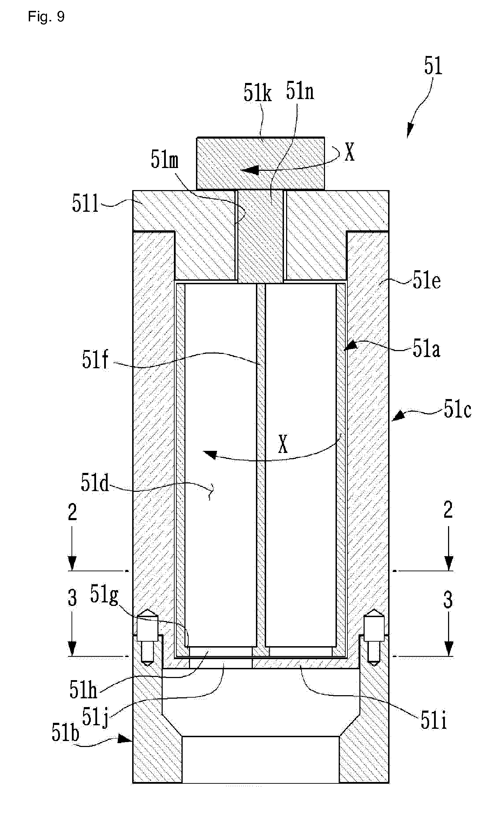

Referring to Figs. 9 to 11, the medication storage chamber 51 of the medication storage part 50 connects to the medication supply tube 52, and includes a medication storage 51a, a medication chamber 51b, a medication selector 51c, and at least two medication cells 51d that are longitudinally separated to receive various medications. The medication storage 51a is provided with a housing 51e. The medication storage 51a has a hollow cylindrical shape, and thus, can be rotated within the housing 51e. An inner partition 51f of the medication storage 51a divides the medication storage 51a into at least two longitudinal spaces. As illustrated in Fig. 10, the partition 51f may have a cross shape, and thus, the number of the medication cells 51d may be four. However, the number of the medication cells 51d is not limited to four, and thus, may be two or more. Outlets 51h are disposed in lower ends 51g of the medication cells 51d, respectively, to discharge a medication.

The medication chamber 51b is disposed under the medication storage 51a to receive a medication discharged through the outlets 51h from the medication cells 51d. That is, the medication chamber 51b is disposed between the medication supply tube 52 and the medication storage 51a to form a space for temporarily storing a medication discharged from the medication cells 51d. The medication chamber 51b may have a hollow cylindrical shape having an outer diameter that is approximately the same as that of the housing 51e to be described later. A medication stored in the medication chamber 51b is discharged to the micro needle head 60 through the medication supply tube 52.

The medication selector 51c is used to select one from the medication cells 51d of the medication storage 51a. Then, a medication stored in the selected one is discharged to the micro needle head 60 through the medication chamber 51b and the medication supply tube 52. The medication selector 51c may be configured in various forms.

For example, the medication selector 51c may be configured to rotate the medication storage 51a for selecting one from the medication cells 51d of the medication storage 51a.

The medication selector 51c includes the housing 51e allowing the rotation of the medication storage 51a therein, and a blocking plate 51i disposed at the lower end of the housing 51e. The lower end of the housing 51e is inserted in the medication chamber 51b. A sealing member (not shown) may be disposed between the blocking plate 51i and the lower ends 51g of the medication storage 51a to allow rotation of the medication storage 51a relative to the blocking plate 51i, and to prevent leakage and mixing of medications stored the medication cells 51d. Referring to Figs. 9 and 11, the blocking plate 51i is provided with a selection hole 51j corresponding to the outlet 51h of one of the medication cells 51d. Thus, when the medication storage 51a is rotated to match the outlet 51h of one of the medication cells 51d with the selection hole 51j, a medication stored in the medication cell 51d is discharged to the medication chamber 51b through the selection hole 51j.

A rotator 51k is disposed on the upper end of the medication storage 51a for a user to rotate the medication storage 51a, and extends from the cover 43 of the power source part 40 although not shown. Thus, a user can conveniently rotate the medication storage 51a.

An upper cap 511 is disposed over the housing 51e to close the upper portion of the medication storage 51a. The upper cap 511 is provided with a support hole 51m to support a support shaft 51n for rotating the rotator 51k. Thus, the support shaft 51n of the rotator 51k can rotate within the support hole 51m of the upper cap 511. When a user holds and rotates the rotator 51k in an arrow direction X as illustrated in Fig. 9, the medication storage 51a is also rotated in the arrow direction X within the housing 51e. Thus, when the rotator 51k is rotated to match the outlet 51h of one of the medication cells 51d with the selection hole 51j of the blocking plate 51i, a medication stored in the medication cell 51d is discharged to the medication chamber 51b through the selection hole 51j.

Furthermore, referring to Figs. 12 and 13, an elastic element 51o and an opening/closing ball 51p may be disposed within each of the medication cells 51d. Thus, a user can select one of the medication cells 51d by just feeling that the opening/closing ball 51p is inserted the selection hole 51j by elasticity of the elastic element 51o disposed within the medication cell 51d, without seeing the insertion of the opening/closing ball 51p with his/her own eyes.

As such, when the elastic elements 51o and the opening/closing balls 51p are disposed within the medication cells 51d, the medication selector 51c includes the housing 51e allowing the rotation of the medication storage 51a therein, and the blocking plate 51i disposed at the lower end of the housing 51e. In this case, the lower end of the housing 51e is inserted in the medication chamber 51b, and the medication storage 51a can be rotated relative to the blocking plate 51i. In addition, the blocking plate 51i is provided with the selection hole 51j corresponding to the outlet 51h of one of the medication cells 51d. Referring to Fig. 13, protrusions 51q are disposed around the selection hole 51j of the blocking plate 51i, and a medication from the medication cell 51d is discharged between the protrusions 51q to the medication chamber 51b.

As described above, when a user holds and rotates the rotator 51k in the arrow direction X as illustrated in Fig. 12, the medication storage 51a is also rotated in the arrow direction X within the housing 51e. Thus, when the rotator 51k is rotated to match the outlet 51h of one of the medication cells 51d with the selection hole 51j of the blocking plate 51i, a medication stored in the medication cell 51d is discharged to the medication chamber 51b between the protrusions 51q disposed around the selection hole 51j.

The micro needle head 60 makes it possible to inject a medication through a skin C without pain, and physically penetrates the epidermis including the corneum of the skin C so as to improve spread speed of the medication through the skin C. Referring to Figs. 14 and 15, the micro needle head 60 includes a fixing body 61, a medication blocking part 63, a needle fixing plate 68, and a needle cover 65.

The fixing body 61 is provided with an inlet 62 coupling to the medication supply tube 52, and a medication passage 66 is disposed in the fixing body 61 to communicate with the medication supply tube 52. The medication passage 66 has an angled gourd shape, and includes an upper passage 66a, a lower passage 66b, and a middle passage 66c for connecting the upper passage 66a to the lower passage 66b. A blocking seat 64a having a truncated conic shape is disposed in the lower portion of the upper passage 66a, and is coupled to a blocking end of a blocking bar 64 to be described later, to block the medication passage 66. A first spring seat 67a is disposed in the upper portion of the lower passage 66b to support the upper end of a spring 67 to be described later. The medication passage 66 is opened as illustrated in Fig. 15 when the micro needle head 60 contacts the skin C, and is closed as illustrated in Fig. 14 when the micro needle head 60 is spaced apart from the skin C. To this end, the medication blocking part 63 is disposed within the medication passage 66.

The medication blocking part 63 includes the blocking bar 64 that is movable between a first position for opening the medication passage 66 (refer to Fig. 15) and a second position for closing the medication passage 66 (refer to Fig. 14). The blocking bar 64 includes a pressing end 64b, a second spring seat 64c, and a blocking end 64d. When the blocking bar 64 is disposed in the second position, the pressing end 64b further protrudes out of the needle cover 65 than the front end of micro needles A does. The second spring seat 64c supports the lower end of the spring 67 such that the spring 67 is installed between the first spring seat 63a and the second spring seat 64c, and has through holes (not shown) therein to pass a medication. The blocking end 64d has a shape corresponding to the blocking seat 64a of the medication passage 66. Thus, when the blocking bar 64 is disposed in the second position, the blocking end 64d engages with the blocking seat 64a to block the medication passage 66.

To maintain the blocking bar 64 in the second position, the spring 67 is disposed between the first spring seat 67a of the upper passage 66a and the second spring seat 64c of the blocking bar 64 to elastically support the blocking bar 64. The spring 67 may include a compression spring.

Accordingly, when the micro needle head 60 contacts the skin C as illustrated in Fig. 15, the pressing end 64a is moved upward against elastic force of the spring 67 by the skin C so as to move the blocking bar 64 to the first position. Thus, the blocking end 64d is spaced apart from the blocking seat 64a to open the medication passage 66. On the contrary, when the micro needle head 60 is spaced apart from the skin C as illustrated in Fig. 14, the blocking bar 64 is returned to the second position by the elastic force of the spring 67. Thus, the blocking end 64d contacts the blocking seat 64a to close the medication passage 66.

The needle fixing plate 68 fixes the upper ends of the micro needles A in an array form. The lower ends of the micro needles A, each of which is solid and not hollow, protrude out of the needle cover 65. Thus, when contacting the skin C, the micro needles A penetrates the epidermis of the skin C, and a medication supplied from the medication passage 66 is transferred to the dermis of the skin C. For example, a protrusion length of the lower ends of the micro needles A protruding out of the needle cover 65 may range from about 200 to 500 µm. Thus, when the micro needles A contact the skin C, the lower ends of the micro needles A penetrate the epidermis of the skin C to efficiently transfer a medication, without stimulating pain spots of the skin C.

The needle cover 65 and the needle fixing plate 68 are fixed to the fixing body 61 by fixing screws 65b so as to form a medication distributing space 65a that communicates with the medication passage 66 between the needle cover 65 and the needle fixing plate 68. Needle holes 65c are disposed in the needle cover 65. The micro needles A fixed to the needle fixing plate 68 pass through the needle holes 65c, respectively, and protrude out of the needle holes 65c.

The needle holes 65c have an inner diameter slightly greater than the outer diameter of the micro needles A.

The size of the medication distributing space 65a and the size of the needle holes 65c are determined to satisfy the following conditions: when the micro needle head 60 contacts the skin C, and the blocking bar 64 opens the medication passage 66, a medication in the medication distributing space 65a is discharged with a certain speed or a certain amount along the micro needles A by capillary action between the micro needles A and the needle holes 65c; and when the micro needle head 60 is spaced apart from the skin C, and the blocking bar 64 closes the medication passage 66, a medication in the medication distributing space 65a is prevented from being discharged along the micro needles A between the micro needles A and the needle holes 65c.

The fixing body 61, the medication blocking part 63, the needle fixing plate 68, and the needle cover 65 are formed of a transparent material such as polymethyl methacrylate (PMMA), so that far infrared rays generated from the far infrared generator 76, and infrared rays generated from the infrared generator 77 can be transmitted to the skin C. Outer circumferential surfaces of the fixing body 1, the needle fixing plate 68, and the needle cover 65 may be coated with a heat resistant paint to improve aesthetic quality.

After a medication is injected, the micro needle head 60 is sealed by a needle head cap 90, and is kept.

As described above, the micro needles A of the micro needle apparatus B are not hollow, and a medication is transferred along the micro needles A from the medication passage 66 through the medication distributing space 65a, and then, is supplied to the skin C through the spaces between the micro needles A and the needle holes 65c. However, the present disclosure is not limited thereto. For example, within the scope of the present disclosure, a micro needle apparatus (not shown) according to an embodiment may include hollow micro needles A, and a medication may be supplied from a medication passage 66 to a skin C through hollow spaces of the micro needles A.

Hereinafter, a micro needle apparatus according to an embodiment will now be described in detail with reference to the accompanying drawings.

Figs. 17 and 18 are views illustrating a micro needle apparatus B' according to an embodiment.

The micro needle apparatus B' includes micro needles A that penetrate the dermis of a skin C to induce cells to naturally heal a wound and to generate collagen, thereby reducing a wrinkle and pigmentation. To this end, the micro needle apparatus B' further includes a micro needle head part 100, an infrared generator part 200, and a power supply part 300. When the micro needle apparatus B' is pressed against the skin C, the front ends of the micro needles A reaching the dermis of the skin C protrude out of the micro needle head part 100. The micro needles A have an outer diameter ranging from about 1 µm to 100 µm, and the front end thereof has an angle ranging from about 37° to 44°.

For example, a protrusion length of the front ends of the micro needles A protruding out of the micro needle head part 100 may range from about 200 µm to 500 µm. Thus, the micro needles A reach the dermis of the skin C through the epidermis thereof, without stimulating pain spots of the skin C. The micro needle head part 100 may be covered with a protective cap 150 to protect the micro needles A when not in use.

The infrared generator part 200 generates infrared rays or far infrared rays (hereinafter, referred to as infrared rays in common) which are emitted to the skin C through the inside or outside of the micro needles A. Infrared rays stimulate atoms, molecules and cells of the human body to activate the cells; remove active oxygen accumulated by environment pollution from the body; and cut off double bonds of unsaturated fatty acid, to thereby improve cosmetic effect. In addition, a tissue acidified by an inflammation can be alkalized. Particularly, since improved blood circulation, thermotherapy effect, ripening effect, self-purification effect, dryness-and-moisture effect, neutralization effect, and resonance effect can be obtained, infrared rays are emitted to follicles of the scalp to prevent hair loss.

The infrared generator part 200 includes an LED or laser diode (LD) as an optical device 210 for generating infrared rays, thereby decreasing the weight and power consumption thereof.

Particularly, unlike a typical micro needle roller illustrated in Fig. 16, the infrared generator part 200 directly emits infrared rays to the skin C through the inside or outside of the micro needles A. That is, an infrared generator of the typical micro needle roller is disposed around micro needles, e.g., at a clamp arm supporting a roller as illustrated in Fig. 16, to function just as a heater for heating and relaxing a skin. However, according to the current embodiment, infrared rays not only heat and relax the skin C, but also directly reach the dermis of the skin C through the inside or outside of the micro needles A penetrating the epidermis of the skin C, thereby maximizing the above described effects of infrared rays.

The power supply part 300 supplies energy required for the infrared generator part 200 to generate an infrared ray. Dry cells 310 (including rechargeable cells), which are portable and conveniently handled, may be used as the power supply part 300. Alternatively, the dry cells 310 may be replaced with an external power source.

The configuration of the micro needle head part 100 will now be described in more detail with reference to Figs. 19 and 20. The micro needle head part 100 includes: a fixing body 104 receiving the infrared generator part 200; a needle fixture 140 adjacent to a side of the fixing body 104 to fix the micro needles A; and a needle cover 142 including needle holes 144 through which the micro needles A are exposed, respectively.

The micro needles A are fixed to the needle fixture 140, and the front ends of the micro needles A are exposed to the outside through the needle holes 144 of the needle cover 142 to which the needle fixture 140 is coupled. In this case, size and coupling position of each component are designed such that the protrusion length of the micro needles A ranges from about 200 µm to 500 µm. Practically, the needle fixture 140 may be inserted and fixed in the needle cover 142 to improve a structural strength and miniaturization.

Each of the needle holes 144 has an inner diameter greater than the outer diameter of the micro needles A such that infrared rays from the infrared generator part 200 are emitted to the skin C through the outside of the micro needles A.

The fixing body 104 is disposed at the opposite side of the needle fixture 140 to the needle cover 142 to receive the infrared generator part 200, and has passages through which infrared rays generated from the received infrared generator part 200 are emitted to the micro needles A and the needle fixture 140. The infrared generator part 200 includes one or more optical devices 210 (LED or LD) for generating an infrared ray and a board 220, and is fitted on a protrusion disposed the top of the fixing body 104. The optical devices 210 are disposed within through holes 106 disposed in the fixing body 104.

Accordingly, the optical devices 210 face the needle fixture 140. Furthermore, a material having excellent reflectivity may be applied to walls of the passages surrounding the optical devices 210 in order to emit a larger amount of infrared rays to the needle fixture 140.

The micro needles A and the needle fixture 140 may be formed of a transparent material for transmitting an infrared ray, to increase infrared rays emitted to the skin C through the insides of the micro needles A, and infrared rays emitted to the skin C through the outsides of the micro needles A. Particularly, in this case, infrared rays emitted through the insides of the micro needles A directly stimulate and activate tissues such as follicles and the dermis under the epidermis, thereby maximizing effects thereof.

A part of the micro needles A are illustrated in Figs. 19 and 20. Referring to Figs. 19 and 20, the micro needles A may include hollow parts 103 functioning as passages along the longitudinal center thereof. Infrared rays emitted from the infrared generator part 200 pass through the passages. Also in this case, the micro needles A and the needle fixture 140 may be formed of a transparent material. Furthermore, since the micro needles A include the hollow parts 103, even when the micro needles A are formed of a material blocking an infrared ray, infrared rays can be emitted through the hollow parts 103.

Examples of the transparent material for transmitting an infrared ray may include polymethyl methacrylate (PMMA), poly carbonate (PC), and glass. However, the transparent material is not limited thereto, and thus, may be any material for transmitting an infrared ray.

A reflective surface 108 may be disposed on at least one portion of the fixing body 104 facing the needle fixture 140, to reflect an infrared ray reflected from the needle fixture 140. A portion of an infrared ray emitted from the infrared generator part 200 is reflected according to an incident angle thereof, from an interface between air and the needle fixture 140, and an interface between the needle fixture 140 and the micro needle A. At this point, the reflective surface 108 disposed in the fixing body 104 reflects infrared rays reflected from the interfaces, so as to increase the amount of infrared rays finally arriving at the skin C.

The reflective surface 108 may be a spherical or parabolic surface, and be concave or convex. The reflective surface 108 illustrated in Figs. 19 and 20, which is concave, collects infrared rays to the center of the needle fixture 140, thereby compensating for a shortage of infrared rays at the center of the needle fixture 140. Otherwise, the amount of infrared rays at the center of the needle fixture 140 would be smaller than the amount of infrared rays at the edge thereof since the optical devices 210 are adjacent to the edge of the fixing body 104. Although not shown, when the reflective surface 108 is convex, a shortage of infrared rays at the edge of the needle fixture 140 can be compensated for. Furthermore, the reflective surface 108 may have a combination of concave and convex surfaces (not shown). The reflective surface 108 may be polished, or be coated with a light reflecting material to improve re-directivity of an infrared ray.

Further, the micro needle apparatus B' may include a switch mechanism that operates the infrared generator part 200 only when the micro needle head part 100 tightly contacts the skin C, thereby improving power efficiency and use convenience.

A first example of the switch mechanism is a switch body 110 accommodated in the fixing body 104. When the micro needle head part 100 contacts the skin C, the switch body 110 electrically connects the power supply part 300 to the infrared generator part 200. When the micro needle head part 100 is spaced apart from the skin C, the switch body 110 electrically disconnects the power supply part 300 and the infrared generator part 200 from each other.

A detailed constitution and operation of the switch body 110 is illustrated in Figs. 19 and 20. The switch body 110 includes: a rod 112 movable between a first position (refer to Fig. 20) for electrically connecting the power supply part 300 to the infrared generator part 200, and a second position (refer to Fig. 19) for electrically disconnecting the power supply part 300 from the infrared generator part 200; and a spring 122 elastically supporting the rod 112 in the second position when the rod 112 is idle. An end of the rod 112 protruding out of the micro needle head part 100 functions as a pressing end 113. Pressure generated when pressing the rod 112 against the skin C is transmitted to the rod 112 by the pressing end 113.

A protrusion length of the pressing end 113 may be greater than that of the micro needles A. Thus, infrared rays are emitted just before the micro needles A penetrate the skin C, thereby improving effects of infrared rays.

The rod 112 can reciprocate between the first and second positions. To this end, for example, the switch body 110 includes a through hole 124 provided with a stepped protrusion 126, and the rod 112 inserted in the through hole 124 includes a first stopper protrusion 114 and a second stopper protrusion 116, which are disposed at the inside and outside of the stepped protrusion 126, respectively. To this end, the rod 112 is constituted by two removable pieces. A preload is applied to the spring 122 between the stepped protrusion 126 and the first stopper protrusion 114, so as to elastically and entirely support the rod 112.

The switch body 110 is integrally coupled to the fixing body 104, and the rod 112 disposed in the central portion of the switch body 110 is exposed to the outside through the fixing body 104, the needle fixture 140, and the needle cover 142. Thus, when pressure is not applied to the pressing end 113, the rod 112 is maintained in the second position that is a maximum protrusion state. In addition, when pressure is applied to the pressing end 113, the rod 112 is moved upward to the first position. Thus, when the rod 112 is in the first position, the rod 112 forms a contact point connecting the power supply part 300 to the infrared generator part 200. Accordingly, only when the pressing end 113 is pressed against the skin C, that is, only when the micro needle apparatus B' is in use, infrared rays are emitted. When the pressure is removed from the pressing end 113, the rod 112 is returned to the second position to remove the contact point, and the infrared radiation is stopped.

When the needle cover 142, the needle fixture 140, the fixing body 104, and the switch body 110 are integrated into the micro needle head part 100 by bolts 146, manufacturing, handling and usage efficiency, and structural strength are improved. Moreover, the integrated micro needle head part 100 may be used as a cover for an inlet of a handle 400 having a barrel shape to accommodate the power supply part 300, which will be described later, thereby improving convenience. The dry cells 310 are taken in and out through the inlet of the handle 400.

A constitution of the contact point, which connects the power supply part 300 to the infrared generator part 200 when the rod 112 is in the first position, will now be described.

A first terminal 118 is disposed on the second stopper protrusion 116 of the rod 112. When the rod 112 is in the first position, the first terminal 118 is connected to a positive (+) terminal of the power supply part 300 to supply positive (+) electricity to the infrared generator part 200. A second terminal 120 is disposed on the switch body 110, and is always connected to a negative (-) terminal of the power supply part 300 to supply negative (-) electricity to the infrared generator part 200. Thus, only when the first terminal 118 disposed on the second stopper protrusion 116 of the rod 112 is connected to the positive (+) terminal of the power supply part 300, the infrared generator part 200 is operated. When the power supply part 300 is constituted by the dry cells 310, the second stopper protrusion 116 may face a positive (+) pole the dry cell 310 such that the first terminal 118 of the second stopper protrusion 116 may directly contact the positive (+) pole of the dry cell 310, thereby simplifying the structure thereof.

In addition, as described above, the micro needle head part 100 may be integrated to be used as a cover for the inlet of the handle 400 having a barrel shape to accommodate the dry cells 310 as the power supply part 300 that are taken in and out through the inlet of the handle 400. Particularly, the switch body 110 may be provided with a male screw plug 128 that is screwed to the handle 400, whereby the micro needle head part 100 can function as the cover. In this case, when the switch body 110 is screwed to the handle 400, the switch body 110 may be electrically connected to the negative (-) terminal of the power supply part 300.

Referring to Figs. 19 and 20, since the second stopper protrusion 116 is disposed within a recess part 130 of the switch body 110, the positive (+) pole of the dry cell 310 is spaced apart from a first electrode of the rod 112 in the second position. In this state, when the rod 112 is moved upward to the first position, the first electrode contacts the positive (+) pole of the dry cell 310 to form a closed circuit connecting the power supply part 300 to the infrared generator part 200. A negative (-) pole of the dry cells 310 may be elastically supported by a conical coil spring or bent metal plate, to ensure electrical contact. Thus, even when the rod 112 moves to the first position, and presses the dry cells 310, excessive stress is prevented from being applied to the rod 112. A buffer 132 may be attached to the top surface of the male screw plug 128, and appropriately press the dry cells 310, thereby preventing the dry cells 310 from being shaken within the handle 400.

According to the current embodiment, the infrared generator part 200 may include LEDs or LDs as the optical devices 210 for generating an infrared ray. In this case, a portion of the LEDs or LDs may be coated with a fluorescent paint that reacts with an infrared ray to emit visible light. Since an infrared ray is invisible light, a user can indirectly recognize an operation of the infrared generator part 200 according to the emission of visible light. Accordingly, the user can easily check a malfunction of the micro needle apparatus B', and the service life of the dry cells 310.

As described above, the switch body 110 senses that the micro needle head part 100 contacts the skin C, to automatically operate the infrared generator part 200. Alternatively, the switch body 110 may be replaced with a switch mechanism that is manually operated by a user.

That is, referring to Fig. 21, a micro needle apparatus according to another embodiment includes a push button switch 410 on a power supply part 300 to selectively supply or cut off energy to an infrared generator part 200. Thus, when a user uses the micro needle apparatus, holing a handle 400, the user can manually operate the infrared generator part 200 by manipulating the push button switch 410.

In this case, the push button switch 410 may be disposed in a position corresponding to a user's thumb as illustrated in Fig. 21. Alternatively, although not shown, the push button switch 410 may be disposed at an end of the handle 400 corresponding to a position indicated by the reference numeral '500' of Fig. 21, so that a finger of a user holding the handle 400 can naturally press the push button switch 410. Components according to the current embodiment are the same as those of the previous embodiment including the micro needle head part 100, except for the push button switch 410 replacing the switch body 110.

Although embodiments have been described with reference to a number of illustrative embodiments thereof, it should be understood that numerous other modifications and embodiments can be devised by those skilled in the art that will fall within the spirit and scope of the principles of this disclosure.

## Claims

1. A micro needle comprising:
a needle comprising an inclined part comprising an inclined outer wall, a straight part comprising a straight outer wall,
and a recess having a certain depth along the inclined or
straight outer wall; and
a body coupled to the needle to move or support the needle.

2. The micro needle according to claim 1, wherein the recess has a straight line shape extending along the needle, and is recessed a certain depth toward a center of the needle, and
the recess is provided in three or more along an outer wall of the needle, or in four with a constant interval along the outer wall at upper, lower, left, and right sides.

3. The micro needle according to claim 1, wherein the recess has a screw thread shape extending clockwise or counterclockwise along an outer wall of the needle.

4. The micro needle according to claim 1, wherein the body has a cylindrical shape and a taper shape that decreases in diameter at a portion connecting to the needle.
